# EUROPEAN PATENT APPLICATION

(11) **EP 3 992 631 A1**
(43) Date of publication of application: **04.05.2022**
(21) Application number: 20205355.9
(22) Date of filing: 03.11.2020
(51) Int. Cl.: G01N 33/574, G01N 33/68

(54) **METHODS OF DIAGNOSING AND/OR PROGNOSING A DISEASE IN TEAR SAMPLE**

(71) Applicant: Ideogen AG, 8640 Hurden (CH)
(72) Inventor: Fidan, Ozan, 06790 Ankara (TR)
(74) Representative: KATZAROV S.A.

(57) **Abstract**

The invention relates to methods of diagnosing, prognosing, stratifying, or determining the progression or regression of a disease, based on the detection of one or more metabolite profile(s) in tears.

## Description

### FIELD OF THE INVENTION

The invention relates to methods of diagnosing and/or prognosing stratifying, determining the progression or regression of a disease based on the detection of one or more metabolite profiles in tears.

### BACKGROUND OF THE INVENTION

Glioblastoma multiforme (GBM) is a highly malignant primary tumor of the central nervous system and most common and lethal of all primary malignant brain tumors (13-16 % of all brain tumors). The current standard therapy is based on oral Temozolomide (TMZ) combination with radiotherapy. Isocitrate dehydrogenase 1 (IDH1) and IDH2 mutations are well known clinical markers of GBM disease. Because of IDH1 and IDH2 mutations, mutated enzymes triggers to produce 2-hydroxyglutarate (2HG) instead of 2-oxoglutarate. There are several LC/MS based 2-HG quantification methods which were validated for gliomas based on human plasma sampling.

Relevance of increased levels of 2-hydroxyglutarate (2-HG) and glioblastoma have been shown by investigators. 2-HG is clinically relevant biomarker not only for glioblastoma but also for other neurological diseases such as, e.g. acute myeloid leukemia. 2-HG quantification in human plasma is well known tool for detection of IDH mutations.2- hydroxyglutarate has 2 enantiomers L(L2HG) and D (D2HG).

However, regarding complexity of human plasma and false-positive and false-negative interpretation of results, 2-HG quantification of GMB patients needs to combine integrative test methods such as next generation sequencing (NGS) technologies and MR

Thus, there is an unmet need for a method of diagnostic and/or prognostic of diseases such as cancer, infectious disease, neurological disease and/or neurodegenerative disease in tears.

### BRIEF DESCRIPTION OF THE FIGURES

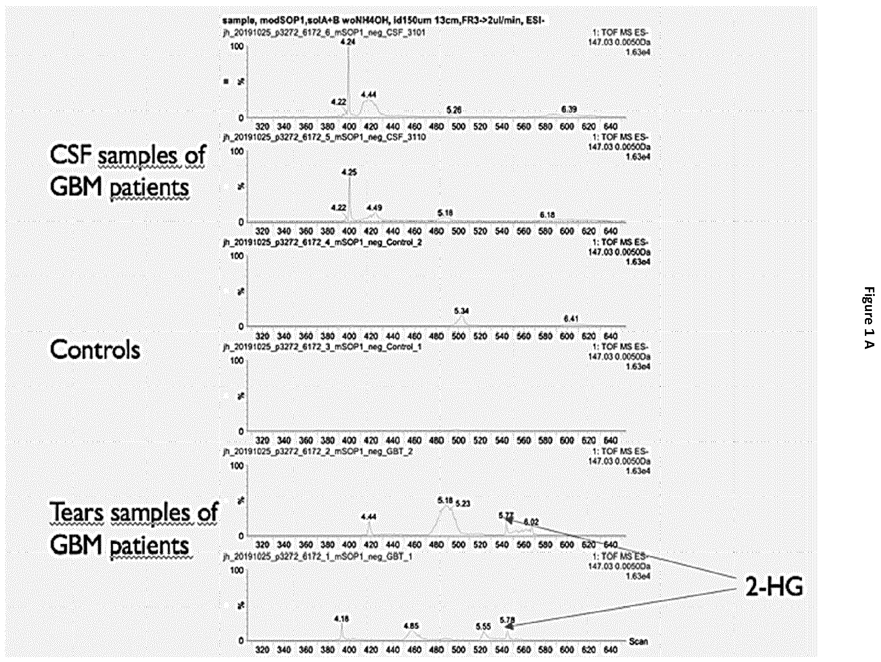

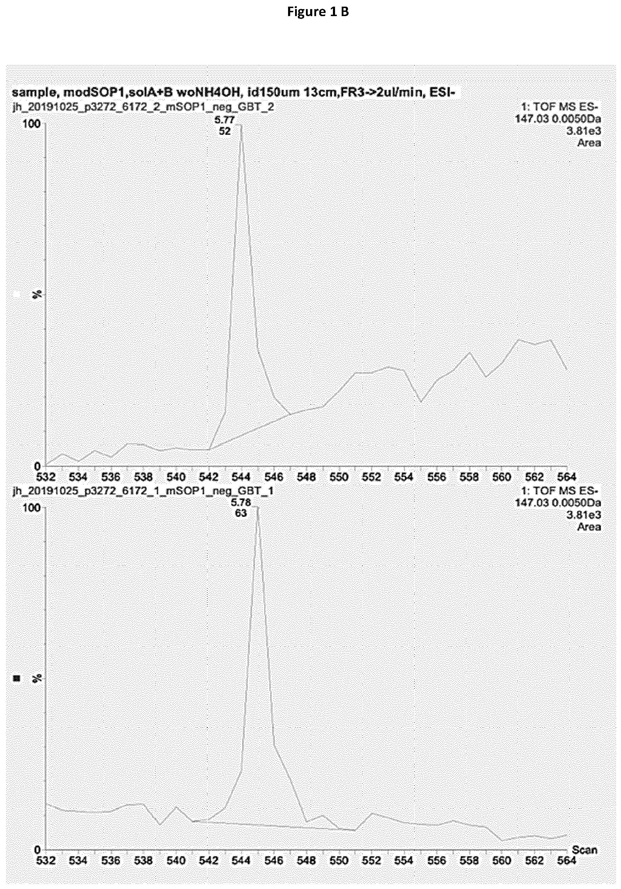

**Figure 1****:** (A) Left graphic, LC-MS chromatogram of CSF samples of GBM patients, Tears samples of Controls and Tears samples of GBM patients which indicates 2-HG existence only in tears of GBM patients. (B) Right graphic, display of 2-HG peaks at LC-MS chromatogram.

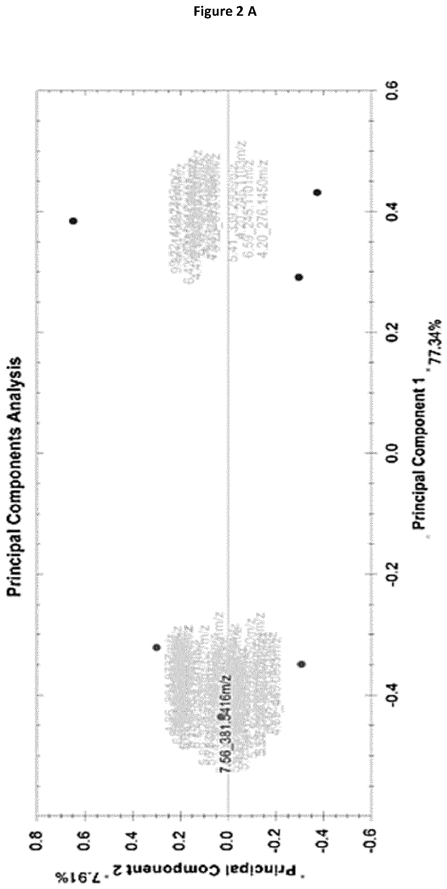

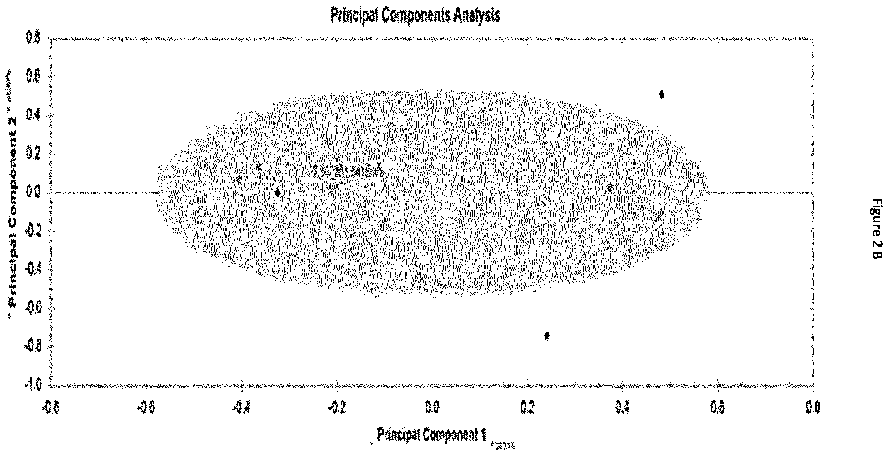

**Figure 2****:** (A) Left graphic, comparison of control samples (3 points at left) and GBM patients' samples (3 points at right) metablomics patterns. (B) Right graphic, comparison of post therapy GBM samples (3 points left), relapse samples of GBM patients (3 points at right).
**Figure 3****:** Experimental data for GBM patients stratification and disease phenotyping as clinical assessment tool

### DESCRIPTION OF THE INVENTION

Although methods and materials similar or equivalent to those described herein can be used in the practice or testing of the present invention, suitable methods and materials are described below. All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

Reference throughout this specification to "one aspect", "an aspect", "another aspect", "a particular aspect", "combinations thereof" means that a particular feature, structure or characteristic described in connection with the invention aspect is included in at least one aspect of the present invention. Thus, the appearances of the foregoing phrases in various places throughout this specification are not necessarily all referring to the same aspect. Furthermore, the particular features, structures, or characteristics may be combined in any suitable manner in one or more aspects.

The term "comprise(s)", or "comprising" is generally used in the sense of include(s)/including, that is to say, permitting the presence of one or more features or components. The terms "comprise(s)" and "comprising" also encompass the more restricted ones "consist(s)" and "consisting", respectively.

As used in the specification and claims, the singular form "a", "an" and "the" include plural references unless the context clearly dictates otherwise.

As used herein, "one or more" metabolite(s) means "at least one" metabolite, e.g. a combination of two, three, four, five, six, *etc...* metabolites.

The term "about", particularly in reference to a given quantity or percentage, is meant to encompass deviations of plus or minus ten (10) percent (+/- 10%).

The term "level," when used in reference to a particular metabolite in a sample in the present application, means an absolute level (e.g. count per second (cps)) or a relative level such as a percent or fraction compared to one or more other molecules in a sample.

The term "amount," when used in reference to a particular metabolite in a sample in the present application, means concentration of said particular metabolite in the sample. In view of the above, it is understood that an amount can be a molar concentration or weight.

Determining both the amount and the level of a metabolite of the invention is also within the scope of the present invention.

In one aspect, the disease is selected from the group comprising, or consisting of, a cancer, an infectious disease, an immune disease, a neurological disease and a neurodegenerative disease. Preferably, the disease is selected from the group comprising, or consisting of, glioblastoma multiforme, acute myeloid leukemia, parkinson's disease, alzheimer's disease, Covid-19, and amyotrophic lateral sclerosis (ALS).

As used herein the terms "subject"/"subject in need thereof", or "patient"/"patient in need thereof" are well-recognized in the art, and, are used interchangeably herein to refer to a mammal, including dog, cat, rat, mouse, monkey, cow, horse, goat, sheep, pig, camel, and, most preferably, a human. In some cases, the subject is a subject in need of treatment or a subject with a disease or disorder. However, in other aspects, the subject can be a normal subject, i.e. a healthy subject. The term does not denote a particular age or sex. Thus, adult and newborn subjects, whether male or female, are intended to be covered. Preferably, the subject is a human, most preferably a human suffering from a disclosed described herein or a human that might be at risk of suffering from a disease disclosed herein.

The present invention is based, in part, on surprising results showing that the presence of one or more metabolites in tears can correlate with the presence, the stratification as well as the progression or regression of a disease.

An aspect of the invention concerns a method of diagnosing a disease in a subject comprising:
(a) detecting the profile of a metabolite in tears obtained from said subject;
(b) comparing the subject's metabolite profile to a healthy control metabolite profile for the same metabolite biomarker; and
(c) identifying differences between the subject's metabolite profile and the healthy control metabolite profile;
wherein the metabolite is 2-hydroxyglutarate (2-HG), or an enantiomer thereof, and wherein a variation in the profile of said 2-hydroxyglutarate (2-HG), or an enantiomer thereof, in the subject's metabolite profile as compared to the healthy control metabolite profile indicates the presence of a disease in the subject.

Any technique and method known in the art for obtaining and collecting tears from a subject are contemplated in the present invention as long as they are fast, non-invasive, inexpensive, easy to use, and with minimal risk of injury to the subject. Non-limiting examples comprise direct and non-direct methods selected from microcapillary tubes (MCT) or micropipettes such glass or polyester fiber rod which will be placed in contact intermittently with the tear fluid, absorbing supports such as Schirmer test strips (STS), filter paper disks, cellulose sponges and polyester rods. Once collected, solvent can optionally be added to the tear samples and they will be either frozen or analyzed before degradation of the metabolites. The solvent will be either polar or non-polar solvent. A preferred polar solvent will be alcohol, most preferably methanol.

Tears can be collected either without previous stimulation (non-stimulated tears) or after previous stimulation or instillation of different volumes of sterile saline (stimulated tears).

Tears can be collected from any part of the eye, in particular they can be collected from one or more of the following part(s): lower fornix, cul-de-sac, upper punctum, plica semilunaris, lower punctum, lateral canthus, and caruncle.

In an aspect of the invention, the tears can be stored frozen (at -20 to -80°C or in liquid nitrogen) theoretically, for years protected from degradation.

The detection of the metabolite profile (i.e. qualitative and/or quantitative tear metabolites) comprises determining the presence, the amount and/or the level of said metabolite. Non-limiting detection and examination methods include one- and two-dimensional gel electrophoresis, ELISA, high performance liquid chromatography (HPLC), mass spectrometry (MS) related techniques such as MS-MS, matrix-assisted laser desorption/ionization time-of-flight MS, surface enhanced laser desorption/ionization time-of-flight MS, Liquid chromatography coupled to mass spectrometry (LC/MS), various antibody arrays, multiplex bead analysis, NMR, Western blot analysis, *etc..*

The metabolite, as disclosed herein, will be selected from the non-limiting group comprising, or consisting of, amino acids, carbohydrates, nucleotides, nucleosides, hormones, organic acids, and any small molecule, any derivative thereof or any combination thereof.

In some aspects of the invention, the metabolite is selected from the group comprising 2-hydroxyglutarate (2-HG) or an enantiomer thereof. Examples of 2-HG enantiomers include D-2-HG and L-2-HG.

2-HG, and in particular its D-enantiomer (D-2-HG) is regarded as an oncometabolite. It is found at elevated levels in plasma in certain malignancies such as acute myeloid leukemia and glioma (e.g. glioblastoma multiforme). It is produced by a mutated isocitrate dehydrogenase IDH1/2, a low-affinity/high-capacity enzyme D-2-HG (Berger, R.S. et al. Degradation of D-2-hydroxyglutarate in the presence of isocitrate dehydrogenase mutations. Sci Rep 9, 7436 (2019).

The methods of the invention comprise the steps of comparing the subject's metabolite profile to a healthy control metabolite profile for the same metabolite biomarker and identifying differences between the subject's metabolite profile and the healthy control metabolite profile.

A variation in the profile of the metabolite, in particular of 2-hydroxyglutarate (2-HG), or one of its enantiomer, in the subject's metabolite profile as compared to the healthy control metabolite profile indicates the presence of a disease in the subject. This variation identification can comprise i) the presence of a metabolite that was not detected in the healthy control metabolite profile, ii) the absence of a metabolite that was detected in the healthy control metabolite profile, iii) an increase or decrease in the amount and/or level of said metabolite when compared to the healthy control metabolite profile.

The increase in the amount and/or level of said metabolite when compared to the healthy control metabolite profile in tears sample according to the methods of the present invention refers, usually, to an increase of equal or superior to about 5 %, preferably equal or superior to about 20 %, more preferably equal or superior to about 40 %, most preferably equal or superior to about 60 %, more preferably equal or superior to about 500%, even more preferably equal or superior to about 1000 %, in particular equal or superior to about 5000 %.

The decrease in the amount and/or level of said metabolite when compared to the healthy control metabolite profile in tears sample according to the methods of the present invention refers, usually, to a decrease of equal or superior to about 5 %, preferably equal or superior to about 20 %, more preferably equal or superior to about 40 %, most preferably equal or superior to about 60 %, more preferably equal or superior to about 500%, even more preferably equal or superior to about 1000 %, in particular equal or superior to about 5000 %.

The present invention also encompasses a method of stratifying a disease in a subject comprising:
(a) detecting the profile of a metabolite in tears obtained from said subject;
(b) comparing the subject's metabolite profile to a healthy control metabolite profile for the same metabolite biomarker; and
(c) identifying differences between the subject's metabolite profile and the healthy control metabolite profile;
wherein the metabolite is 2-hydroxyglutarate (2-HG), or an enantiomer thereof, and wherein a variation in the profile of the level of said 2-hydroxyglutarate (2-HG), or an enantiomer thereof, in the subject's metabolite profile as compared to the healthy control metabolite profile is indicative of the disease stage or grade.

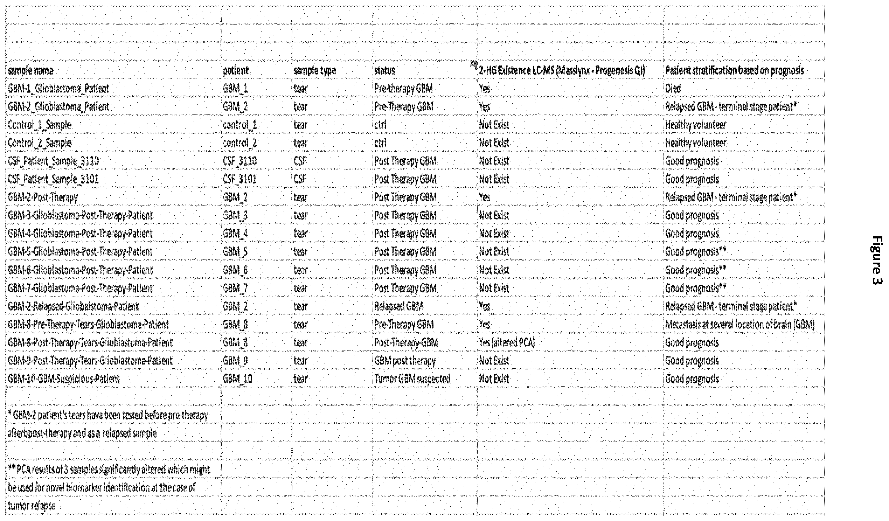

According to comparison of targeted metabolomics profile of pretherapy and post therapy patients, existence of 2-HG has been investigated at relapsed GBM patients correlatively pre-therapy GBM patients. In terms of disease stratification of post therapy GBM patients, as divided into post therapy GBM patients & relapsed GBM patients, significant discrimination at principal component analysis has been observed. Patient stratification analysis was performed as tears phenotyping at several stages GBM patients between February - March 2020 (Fig. 3).

Also encompassed is a method of determining the progression or regression of a disease in a subject suffering from said disease, said method comprising
(a) detecting the profile of a metabolite in tears obtained from said subject;
(b) periodically determining the profile of said metabolite,
   wherein the metabolite is 2-hydroxyglutarate (2-HG), or an enantiomer thereof, and
   wherein an alteration in the profile of said 2-hydroxyglutarate (2-HG), or an enantiomer thereof, in said tears, relative to the profile of said 2-hydroxyglutarate (2-HG), or an enantiomer thereof, determined previously, is indicative of the progression or regression of said disease.

According to targeted metabolomics data from several stages of GBM patients (progression investigation via targeted metabolomics research / 2-HG);
- 2-HG existence were found within chromatogram of tears of pre-therapy GBM samples.
- 2-HG was not found within chromatogram of tears of post-therapy GBM samples.
- 2-HG existence was found within chromatogram of tears of relapsed GBM samples. (Recurrent tumor existence at relapsed GBM patients in comply with LC-MS profiling of tears)

In an aspect of the invention, any method described herein is a computer-implemented method. The computer may include a user interface wherein said user interface relates the presence, the amount and/or the level of said one or more metabolite(s), to detecting a disease in said subject.

An assay for use in a method of anyone of the preceding claims, comprising means and/or reagents for determining the profile of 2-HG, or an enantiomer thereof, in tears of a subj ect.

A kit for performing a method according to any one of claims 1 to 7, said kit comprising
a) means for collecting tears of a subject,
b) means and/or reagents for determining the profile of 2-HG, or an enantiomer thereof, in said tears, and
c) instructions for use.

The present invention further encompasses a device for performing a method of the invention, said device comprising
a) a sample chamber for holding tears collected from a subject,
b) an assay module in fluid communication with said sample chamber, said assay module comprising means and/or reagents for determining the profile one or more metabolite(s), in said tears,
   wherein said assay module comprises:
   a user interface wherein said user interface relates the presence, the amount and/or the level of said one or more metabolite(s), in said assay module to detecting a disease in said subject.

The disclosure is further illustrated by the following examples. The examples below are non-limiting and are merely representative of various aspects of the disclosure.

### EXAMPLES

### Material & Methods

### Analysis of metabolites in serum and tissue

### Metabolite extraction from tears

50 µL of tears were mixed with 200 µl of methanol (HPLC grade) in a 1.5ml Eppendorf tube. The mixture was vortexed for 10 s, and then incubated for 20 min on ice. After centrifugation (16,000 x g, 4 °C, 15 min), 200 µl of the supernatant containing the extracted metabolites was transferred to a 1 ml glass vial and stored at -20 °C until analysis.

### Sample preparation for LC-MS analysis

50 µL methanol extract was dried under a nitrogen stream and reconstituted in 20uL water (MS grade) and diluted with 80uL injection buffer. The dilution was vortexed and centrifuged (16,000 x g, 4 °C, 15 min). 50 µL of the supernatant was transferred to a glass vial with narrowed bottom (Total Recovery Vials, Waters) for LC-MS injection. In addition, method blanks, QC standards, and pooled samples were prepared in the same way to serve as quality control for the measurements. Injection buffer was composed of 90 parts of acetonitrile (HPLC grade), 9 parts of methanol (HPLC grade) and 1 part of 5M ammonium acetate (p.a.).

### LC-MS analysis

Metabolites were separated on a nanoAcquity UPLC (Waters) equipped with a BEH Amide capillary column (150 µm x50mm, 1.7 µm particle size, Waters), applying a gradient of 5mM ammonium acetate in water (A) and 5mM ammonium acetate in acetonitrile (B) from 5% A to 50% A over 12min. The injection volume was 1 µL. The flow rate was adjusted over the gradient from 3 to 2 µl/min.

The UPLC was coupled to Synapt G2Si mass spectrometer (Waters) by a nanoESI source. MS1 (molecular ion) and MS2 (fragment) data was acquired using negative polarization and MS^{E} over a mass range of 50 to 1200 m/z at MS1 and MS2 resolution of 25'000 FWHM

### Targeted Data analysis

Target metabolites (2-HG) were quantified by the area under the peak (AUP) of the MS 1 extracted ion chromatogram (EIC) of the respective [M-H]⁻ ion by using MassLynx v4.2 software (Waters). Reference samples were used to locate and verify the correct peaks on the EIC of 2-HG by retention time and MS2 fragment information. AUP values reported are unit less and are proportional to the concentration of the targets in the samples (relative quantification). AUP values can be used to calculate fold changes.

### Untargeted Metabolomics Data analysis

Metabolomics data sets were evaluated in an untargeted fashion with Progenesis QI software (Nonlinear Dynamics), which aligns the ion intensity maps based on a reference data set, followed by a peak picking on an aggregated ion intensity map. Detected ions were identified based on accurate mass, detected adduct patterns and isotope patterns by comparing with entries in the Human Metabolom Data Base (HMDB). A mass accuracy tolerance of 5mDa was set for the searches. Observed fragmentation patterns were considered for the identifications of metabolites by comparison to theoretical fragmentation spectra. All biological samples were analysed in triplicate and quality controls were run on pooled samples and reference compound mixtures to determine technical accuracy and stability.

### Results

### Targeted metabolomics analysis of tears of GBM patients (GBM patients phenotyping)

2-HG was found LC-MS chromatogram of tear samples of pre-therapy GBM patients. 2 independent programs were used to identify existence of 2-HG; MassLynx and Progenesis QI (multivariate data analysis). However, 2-HG was not found neither in samples of control groups nor in samples of post-therapy samples of GBM patients.

Furthermore, 2-HG was found within samples of relapsed GBM patients even recurrence of tumor couldn't have been detected by imaging systems such as MR

We have investigated that PCA analysis of GBM patients represents discriminated groups at chromatograms which might be used for phenotyping of GBM patients.

When we compared the PCA of relapsed GBM patients and good-prognosis patients, we have recognized difference between patterns of untargeted metabolomics which might be used as a prognostic marker.

## Claims

1. A method of diagnosing a disease in a subject comprising:
(a) detecting the profile of a metabolite in tears obtained from said subject;
(b) comparing the subject's metabolite profile to a healthy control metabolite profile for the same metabolite biomarker; and
(c) identifying differences between the subject's metabolite profile and the healthy control metabolite profile;
wherein the metabolite is 2-hydroxyglutarate (2-HG), or an enantiomer thereof, and
wherein a variation in the profile of said 2-hydroxyglutarate (2-HG), or an enantiomer thereof, in the subject's metabolite profile as compared to the healthy control metabolite profile indicates the presence of a disease in the subject.

2. A method of stratifying a disease in a subject comprising:
(a) detecting the profile of a metabolite in tears obtained from said subject;
(b) comparing the subject's metabolite profile to a healthy control metabolite profile for the same metabolite biomarker; and
(c) identifying differences between the subject's metabolite profile and the healthy control metabolite profile;
wherein the metabolite is 2-hydroxyglutarate (2-HG), or an enantiomer thereof, and
wherein a variation in the profile of the level of said 2-hydroxyglutarate (2-HG), or an enantiomer thereof, in the subject's metabolite profile as compared to the healthy control metabolite profile is indicative of the disease stage.

3. A method of determining the progression or regression of a disease in a subject suffering from said disease, said method comprising
(a) detecting the profile of a metabolite in tears obtained from said subject;
(b) periodically determining the profile of said metabolite,
wherein the metabolite is 2-hydroxyglutarate (2-HG), or an enantiomer thereof,, and
wherein an alteration in the profile of said 2-hydroxyglutarate (2-HG), or an enantiomer thereof, in said tears, relative to the profile of said 2-hydroxyglutarate (2-HG), or an enantiomer thereof, determined previously, is indicative of the progression or regression of said disease.

4. The method of anyone of the preceding claims, wherein detecting the profile of a metabolite in tears comprises determining the presence, the amount and/or the level of said metabolite.

5. The method of anyone of the preceding claims, wherein the disease is selected from the group comprising a cancer, an infectious disease, a neurological disease and/or a neurodegenerative disease.

6. The method of claim 5, wherein the disease is selected from the group comprising glioblastoma multiforme, acute myeloid leukemia, parkinson's disease, alzheimer's disease, amyotrophic lateral sclerosis (ALS).

7. The method of anyone of the preceding claims, wherein the 2-HG enantiomer is selected from the group comprising L-2HG and D-2-HG.

8. An assay for use in a method of anyone of the preceding claims, comprising means and/or reagents for determining the profile of 2-HG, or an enantiomer thereof, in tears of a subj ect.

9. A kit for performing a method according to any one of claims 1 to 7, said kit comprising
a) means for collecting tears of a subject,
b) means and/or reagents for determining the profile of 2-HG, or an enantiomer thereof, in tears of a subject, and
c) instructions for use.

10. A method of diagnosing and/or prognosing a disease in a subject comprising:
(a) detecting the profile of one or more metabolite(s) and/or metabolomic pattern in tears obtained from said subject;
(b) comparing the one or more subject's metabolite profile(s) and/or metabolomic pattern to healthy control metabolite profile(s) and/or metabolomic pattern for the same metabolite biomarker(s); and
(d) identifying differences between the subject's metabolite profile(s) and/or metabolomic pattern and the healthy control metabolite profile(s);
wherein an increase or decrease in the profile of said one or more metabolite(s) and/or metabolomic pattern in the subject's metabolite profile(s) as compared to the healthy control metabolite profile(s) indicates the presence of a disease in the subject.

11. The method of claim 10, wherein the disease is selected from the group comprising a cancer, an infectious disease, an immune disease, a neurological disease and a neurodegenerative disease.

12. The method of claim 10, wherein the disease is selected from the group comprising glioblastoma multiforme, acute myeloid leukemia, parkinson's disease, alzheimer's disease, Covid-19, and amyotrophic lateral sclerosis (ALS).

13. The method of claim 10 or 11, wherein the metabolite is an oncometabolite.

14. The method of any one of claims 1 to 7 or claims 10 to 13, wherein said method is a computer-implemented method.

15. A device for performing a method according to any one of claims 1 to 7 or claims 10 to 13, said device comprising
a) a sample chamber for holding tears collected from a subject,
b) an assay module in fluid communication with said sample chamber, said assay module comprising means and/or reagents for determining the profile one or more metabolite(s), in said tears,
wherein said assay module comprises:
a user interface wherein said user interface relates the presence, the amount and/or the level of said one or more metabolite(s), in said assay module to detecting a disease in said subject.
